# EUROPÄISCHE PATENTSCHRIFT

(11) **EP 3 769 149 B1**
(45) Veröffentlichungstag und Bekanntmachung des Hinweises auf die Patenterteilung: **23.02.2022**
(21) Anmeldenummer: 19709687.8
(22) Anmeldetag: 06.03.2019
(51) Int. Cl.: G02B 27/62, G01B 11/27, G01M 11/02

(54) **VERFAHREN ZUM ZENTRIEREN EINES OPTISCHEN ELEMENTS IN EINEM OPTISCHEN SYSTEM FÜR EIN ENDOSKOP**
METHOD FOR CENTERING AN OPTICAL ELEMENT IN AN OPTICAL SYSTEM FOR AN ENDOSCOPE
PROCÉDÉ DE CENTRAGE D'UN ÉLÉMENT OPTIQUE DANS UN SYSTÈME OPTIQUE D'UN ENDOSCOPE

(30) Priorität: 20.03.2018 DE 102018106468
(43) Veröffentlichungstag der Anmeldung: 27.01.2021
(73) Patentinhaber: OLYMPUS WINTER & IBE GMBH, 22045 Hamburg (DE); Fraunhofer-Gesellschaft zur Förderung der angewandten Forschung e.V., 80686 München (DE)
(72) Erfinder: SCHÖLER, Uwe, 22955 Hoisdorf (DE); BECKERT, Erik, 99310 Arnstadt (DE)
(74) Vertreter: Seemann & Partner Patentanwälte mbB
(86) Internationale Anmeldenummer: PCT/EP2019/055535
(87) Internationale Veröffentlichungsnummer: WO 2019/179767

(56) Entgegenhaltungen:
- WO-A1-2010/111465
- DE-A1-102005 003 595
- DE-B- 1 034 637

## Beschreibung

Die Erfindung betrifft ein Verfahren zum Vorbereiten eines optischen Elements zum Einsatz in ein optisches System eines Endoskops und ein Verfahren zum Zentrieren eines optischen Elements in einem optischen System eines Endoskops. Weiterhin betrifft die Erfindung eine Zentrierdrehvorrichtung.

Beim Einsatz von Endoskopen ist eine gute Bildqualität notwendig, um beispielsweise operierenden Ärzten eine gute Sicht auf das Operationsfeld zu ermöglichen. Bei Videoendoskopen erfordert dies den Einsatz von Bildsensoren mit immer höherer Auflösung. Um diese höhere Auflösung zu nutzen, müssen auch die optischen Elemente, beispielsweise die Linsen, sowie ihre Ausrichtung zueinander im Endoskop hohen Anforderungen genügen. So müssen die optischen Achsen aller optischen Elemente im optischen System des Endoskops möglichst exakt mit der optischen Achse des optischen Systems übereinstimmen. Ist hingegen die Abweichung der optischen Achse eines optischen Elements von der optischen Achse des optischen Systems zu groß, sinkt die Bildqualität des Endoskops.

Um die optischen Achsen der optischen Elemente mit der optischen Achse des optischen Systems in Deckung zu bringen, werden die optischen Elemente üblicherweise mittels eines Schleifverfahrens bearbeitet. Dadurch können optische Elemente mit einer Toleranz des Durchmessers von etwa 20 µm bereitgestellt werden. Durch Einsetzen dieser optischen Elemente in eine ebenfalls mit hoher Präzision hergestellte Optikfassung des optischen Systems werden die optischen Elemente entlang der gemeinsamen optischen Achse ausgerichtet.

Mittels eines solchen Schleifverfahrens können jedoch nicht die Toleranzen erreicht werden, die beim Einsatz von hochauflösenden Bildsensoren erforderlich sind. Stattdessen werden in diesem Fall bislang die optischen Elemente in einer Messinghülse eingefasst, die anschließend mit einer speziellen Zentrierdrehmaschine abgedreht wird. Auf diese Weise werden deutlich kleinere Toleranzen des Durchmessers erreicht.

Durch das Einfassen in die Messinghülse wird der Durchmesser des optischen Elements um etwa 200 µm bis 300 µm erhöht, was im Design gegebenenfalls eine Vergrößerung des Endoskopdurchmessers oder eine Verringerung des Durchmessers des optischen Elements erforderlich macht. Zudem kann es bei dem Prozess zu Beschädigungen, beispielsweise Aussprengungen, in der abgedrehten Fläche der Hülse kommen. Schließlich ist das Zentrierdrehen zeitintensiv und das Einbetten des optischen Elements in eine Messinghülse verursacht zusätzliche Kosten. Relevanter Stand der Technik findet sich in den Druckschriften DE 10 34 637 B, WO 2010/111465 A1 sowie DE 10 2005 003595 A1. Die Druckschrift DE 10 2005 003595 A1 erwähnt die Möglichkeit der Bearbeitung optisch aktiver Flächen mittels Laserablation.

Die Aufgabe der Erfindung besteht darin, ein verbessertes Verfahren zum Vorbereiten eines optischen Elements zum Einsetzen in ein optisches System eines Endoskops, ein verbessertes Verfahren zum Zentrieren eines optischen Elements in einem optischen System eines Endoskops, eine verbesserte Zentrierdrehvorrichtung und ein verbessertes Endoskop bereitzustellen.

Diese Aufgabe wird gelöst durch ein Verfahren zum Vorbereiten eines optischen Elements zum Einsatz in ein optisches System eines Endoskops, wobei das optische Element eine optische Achse und eine zur optischen Achse im Wesentlichen parallele Mantelfläche aufweist, mit den folgenden Verfahrensschritten:
- Anordnen des optischen Elements in einer Halterung einer Spindel, die das optische Element um eine Drehachse der Spindel rotiert,
- Ausrichten des optischen Elements auf der Spindel, so dass die optische Achse des optischen Elements mit der Drehachse der Spindel übereinstimmt,
- Abtragen eines äußeren Mantelflächenbereichs des optischen Elements, bis die Mantelfläche einen konstanten Abstand zur optischen Achse des optischen Elements aufweist,
wobei das Verfahren dadurch weitergebildet ist, dass das Abtragen des äußeren Mantelflächenbereichs durch Laserablation, insbesondere mittels eines Ultrakurzpulslasers, erfolgt.

Im Gegensatz zu einem Zentrierdrehverfahren nach dem Stand der Technik wird der üblicherweise verwendete Drehmeißel durch einen Ultrakurzpulslaser ersetzt. Dieser Ultrakurzpulslaser hat vorteilhaft eine Pulsdauer im Femtosekundenbereich. Mit einem derartigen Laser wird das abgetragene Material präziser abgetragen als mit einem Drehmeißel. Weiterhin werden Aussprengungen beim Drehvorgang vermieden. Da der Ablationsvorgang auf atomarer Ebene stattfindet, kann eine Oberfläche mit sehr geringer Rauigkeit erzeugt werden. Das Anfasen erfolgt ebenfalls sehr präzise und reproduzierbar und ohne Aussprengungen.

Bevorzugt wird das optische Element aus einem optisch durchlässigen Material gefertigt, wobei beim Abtragen des äußeren Mantelflächenbereichs direkt das optisch durchlässige Material abgetragen wird. Während es beim Zentrierdrehen mit einem Drehmeißel zu Aussprengungen kommen kann oder eine Platz einnehmende Hülse eingesetzt werden muss, ist dies bei einer Abtragung mittels Laserablation nicht notwendig.

Vorteilhaft wird das optische Element vollständig aus dem optisch durchlässigen Material, beispielweise optischem Glas, gefertigt und es kann auf eine Hülse verzichtet werden. Dies erlaubt die Herstellung von optischen Elementen mit einem größeren Durchmesser als bisher, wodurch mehr Licht eingefangen wird und die Bildqualität eines Endoskops mit diesem optischen Element verbessert wird. Weiterhin entfallen die Kosten, die durch die Einbettung des optischen Elements in eine Hülse entstehen. Alternativ kann der Durchmesser der Optik insgesamt verkleinert werden bei gleichbleibender Lichtmenge, da kein Platz mehr von Messinghülsen verbraucht wird.

In einer bevorzugten Ausführungsform erfolgt das Ausrichten des optischen Elements auf der Spindel mittels wenigstens einer Stoßvorrichtung, wobei die Stoßvorrichtung die Ausrichtung der optischen Achse des optischen Elements bezüglich der Drehachse der Spindel durch Stöße mit einer mit der Halterung verbundenen Ausrichteeinheit verändert.

Die Ausrichteeinheit ist ein Bestandteil der Spindel. Ein erster Teil der Ausrichteeinheit ist so angeordnet, dass seine Ausrichtung gegenüber der Drehachse der Spindel fest ist. Ein zweiter Teil der Ausrichteeinheit, der gelagert auf dem ersten Teil der Ausrichteeinheit angeordnet ist, ist direkt mit der Halterung verbunden. Die Ausrichtung dieses zweiten Teils der Ausrichteeinheit gegenüber der Drehachse der Spindel ist variabel, so dass durch Stöße der Stoßvorrichtung mit dem zweiten Teil der Ausrichteeinheit die Ausrichtung der optischen Achse eines in der Halterung befindlichen Elements gegenüber der Drehachse der Spindel verändert wird. So kann die optische Achse des optischen Elements äußerst präzise gegenüber der Drehachse der Spindel eingestellt werden.

Weiterhin bevorzugt wird das Ausrichten des optischen Elements auf der Spindel durch Erfassung eines Lichtsignals auf einem fotosensitiven Element einer Signalerfassungsvorrichtung überprüft, wobei das Lichtsignal erzeugt wird, indem ein Lichtstrahl durch das optische Element entlang der Drehachse der Spindel in Richtung des fotosensitiven Elements geleitet wird, wobei die Ausrichtung des optischen Elements so lange verändert wird, bis bei einer Drehung der Spindel eine Position des Lichtsignals auf dem fotosensitiven Element konstant bleibt.

Weicht die Ausrichtung der optischen Achse des optischen Elements von der Drehachse der Spindel ab, so beschreibt ein entlang der Drehachse der Spindel geleiteter Lichtstrahl auf dem fotosensitiven Element eine Rotationsbewegung. Mit der Signalerfassungsvorrichtung wird in diesem Fall eine kreisförmige Bewegung des erfassten Lichtsignals aufgezeichnet. Liegt die optische Achse des optischen Elements hingegen genau auf der Drehachse der Spindel, so bleibt die Position des Lichtstrahls auf dem fotosensitiven Element konstant. Auf diese Weise kann sehr genau überprüft werden, ob die optische Achse des optischen Elements mit der Drehachse der Spindel übereinstimmt.

Weiterhin wird die Aufgabe gelöst durch ein Verfahren zum Zentrieren eines optischen Elements in einem optischen System eines Endoskops, wobei das optische System eine optische Achse aufweist und das optische Element eine optische Achse und eine zur optischen Achse im Wesentlichen parallele Mantelfläche aufweist, mit den folgenden Verfahrensschritten:
- Abtragen eines äußeren Mantelflächenbereichs des optischen Elements, bis die Mantelfläche einen konstanten Abstand zur optischen Achse des optischen Elements aufweist,
- Einsetzen des optischen Elements in das optische System, wobei die Mantelfläche des optischen Elements an einer Innenfläche einer Optikfassung des optischen Systems anliegt und die optische Achse des optischen Elements mit der optischen Achse des optischen Systems übereinstimmt,
das dadurch weitergebildet ist, dass das Abtragen des äußeren Mantelflächenbereichs des optischen Elements mittels Laserablation erfolgt, insbesondere mittels des zuvor dargelegten Verfahrens zum Vorbereiten eines optischen Elements zum Einsatz in ein optisches System eines Endoskops.

Gemäß diesem Verfahren zum Zentrieren eines optischen Elements in einem optischen System eines Endoskops ist das optische Element in die Optikfassung des optischen Systems einsetzbar, ohne dass eine Einbettung des optischen Elements in eine Hülse erforderlich wäre. Die Innenfläche der Optikfassung weist einen Durchmesser auf, der im Wesentlichen dem Durchmesser des optischen Elements entspricht. Da die Mantelfläche des optischen Elements nach dem Abtragen des äußeren Mantelflächenbereichs einen konstanten Abstand zur optischen Achse des optischen Elements aufweist, wird durch das Einsetzen des optischen Elements in die Optikfassung des optischen Systems das optische Element so zentriert, dass die optische Achse des optischen Elements mit der optischen Achse des optischen Systems übereinstimmt.

Bevorzugt werden mehrere optische Elemente in das optische System eingesetzt, wobei die optischen Achsen aller optischen Elemente mit der optischen Achse des optischen Systems in Übereinstimmung gebracht werden und wobei die Mantelflächen aller optischen Elemente an der Innenfläche der Optikfassung des optischen Systems anliegen.

Indem mehrere optische Elemente, vorzugsweise alle optischen Elemente des optischen Systems, die eine optische Achse aufweisen, auf die beschriebene Weise bearbeitet und in das optische System eingesetzt werden, wird eine äußerst präzise Ausrichtung der optischen Achsen der optischen Elemente erreicht und eine hohe Bildqualität des Endoskops erzielt.

Das Verfahren zum Zentrieren eines optischen Elements in einem optischen System eines Endoskops verkörpert zudem die gleichen Vorteile, Merkmale und Eigenschaften, wie das zuvor beschriebene Verfahren zum Vorbereiten eines optischen Elements zum Einsatz in ein optisches Systems eines Endoskops.

Schließlich wird die Aufgabe gelöst durch eine Zentrierdrehvorrichtung, umfassend eine um eine Drehachse rotierbare Spindel mit einer Halterung für ein optisches Element für ein Endoskop und eine Abtragungsvorrichtung mit einem Abtragungselement, das dazu eingerichtet ist, eine Mantelfläche eines optischen Elements in der Halterung abzutragen, wobei die Zentrierdrehvorrichtung dadurch weitergebildet ist, dass das Abtragungselement ein Ultrakurzpulslaser ist.

Bei einer Zentrierdrehvorrichtung gemäß dem Stand der Technik wird als Abtragungselement für gewöhnlich ein Drehmeißel verwendet. Dieser wird vorteilhaft durch einen Ultrakurzpulslaser ersetzt.

In einer bevorzugten Ausführungsform ist eine Stoßvorrichtung umfasst, die dazu eingerichtet ist, durch Stöße mit einer mit der Halterung verbundenen Ausrichteeinheit die Ausrichtung der Halterung bezüglich der Drehachse zu verändern.

Weiterhin bevorzugt ist wenigstens eine Signalerfassungsvorrichtung mit einem fotosensitiven Element umfasst, wobei das fotosensitive Element so angeordnet ist, dass durch die Signalerfassungsvorrichtung ein Lichtsignal eines entlang der Drehachse der Spindel geleiteten Lichtstrahls erfassbar ist.

Ebenfalls bevorzugt ist es, wenn die Spindel eine hydrostatische Lagerung aufweist. Vorteilhaft erfolgt die Rotation der Spindel durch die hydrostatische Lagerung äußerst präzise, so dass eine Änderung der Drehachse während der Rotation vermieden wird.

Die Zentrierdrehvorrichtung verkörpert ebenfalls die gleichen Vorteile, Merkmale und Eigenschaften wie die zuvor beschriebenen Verfahren.

Die Aufgabe wird ferner gelöst durch ein Endoskop mit einem optischen Element zum Einsatz in ein optisches System des Endoskops, wobei das optische Element eine optische Achse und eine zur optischen Achse im Wesentlichen parallele Mantelfläche aufweist und wobei das optische Element mit einem Verfahren zum Vorbereiten eines optischen Elements nach einer oder mehreren der zuvor genannten Ausführungsformen vorbereitet ist.

Ferner wird die Aufgabe gelöst durch ein Endoskop mit einem optischen Element zum Einsatz in ein optisches System des Endoskops, wobei das optische Element eine optische Achse und eine zur optischen Achse im Wesentlichen parallele Mantelfläche aufweist und wobei das optische Element mit einem Verfahren zum Zentrieren eines optischen Elements nach einer oder mehreren der zuvor genannten Ausführungsformen zentriert ist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines optischen Elements in einem optischen System gemäß dem Stand der Technik,
- Fig. 2: eine schematische Querschnittsdarstellung eines optischen Elements vor der Durchführung des erfindungsgemäßen Verfahrens,
- Fig. 3: eine schematische Darstellung einer Zentrierdrehvorrichtung,
- Fig. 4a: eine schematische Darstellung eines optischen Elements auf einer Halterung einer Spindel,
- Fig. 4b: eine schematische Darstellung eines fotosensitiven Elements mit einem Lichtsignal,
- Fig. 5a: eine schematische Darstellung des optischen Elements auf der Halterung aus Fig. 4a, mit übereinstimmender Ausrichtung der optischen Achse des optischen Elements und der Drehachse der Spindel,
- Fig. 5b: eine schematische Darstellung eines fotosensitiven Elements mit einem Lichtsignal,
- Fig. 6: eine schematische Darstellung des optischen Elements auf der Halterung gemäß Fig. 5a während einer Laserablation und
- Fig. 7: zwei optische Elemente, die in eine Optikfassung eines optischen Systems eingesetzt wurden.

In den Zeichnungen sind jeweils gleiche oder gleichartige Elemente und/oder Teile mit denselben Bezugsziffern versehen, so dass von einer erneuten Vorstellung jeweils abgesehen wird.

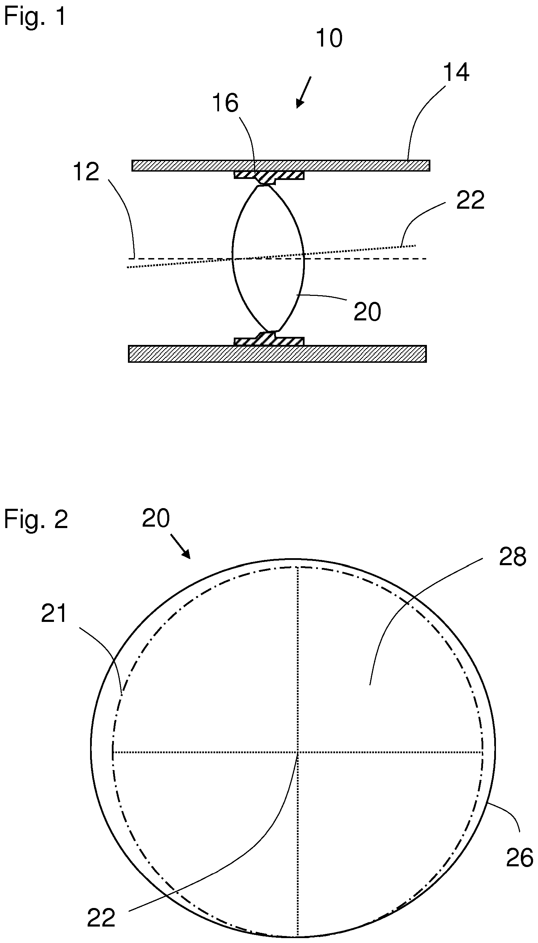

Fig. 1 zeigt schematisch ein optisches System 10 eines Endoskops nach dem Stand der Technik im Querschnitt. In einer Optikfassung 14 des optischen Systems 10 ist ein optisches Element 20 eingesetzt. Das optische Element 20 ist in eine Hülse 16, beispielsweise eine Messinghülse, eingebettet. Da die mechanische Achse der Messinghülse 16 und die optische Achse 22 des optischen Elements 20 nicht übereinstimmen, stimmen auch die optische Achse 12 des optischen Systems 10 und die optische Achse 22 des optischen Elements 20 nicht überein. Um dieses Problem zu lösen, wird gemäß dem Stand der Technik die Messinghülse 16 durch Zentrierdrehen so bearbeitet, dass die optischen Achsen 12 und 22 übereinstimmen. Jedoch ist das Zentrierdrehverfahren sehr arbeitsaufwändig und setzt die Verwendung einer Hülse 16 voraus, wodurch der maximale Durchmesser des optischen Elements 20 begrenzt wird.

Fig. 2 zeigt schematisch ein optisches Element 20 in einer Querschnittsdarstellung. Das optische Element 20 ist aus einem optisch durchlässigen Material 28 gefertigt, beispielsweise optischem Glas. Die Form des optischen Elements 20 weicht von der Form eines Kreises 21 ab. Der Kreis 21 in Fig. 2 ist der größte Kreis, dessen Mittelpunkt die optische Achse 22 bildet und noch vom optischen Element 20 umfasst wird.

Gemäß dem erfindungsgemäßen Verfahren zum Vorbereiten eines optischen Elements zum Einsatz in ein optisches System eines Endoskops wird die Mantelfläche 26 des optischen Elements so abgetragen, dass die Mantelfläche 26 dem Kreis 21 folgt.

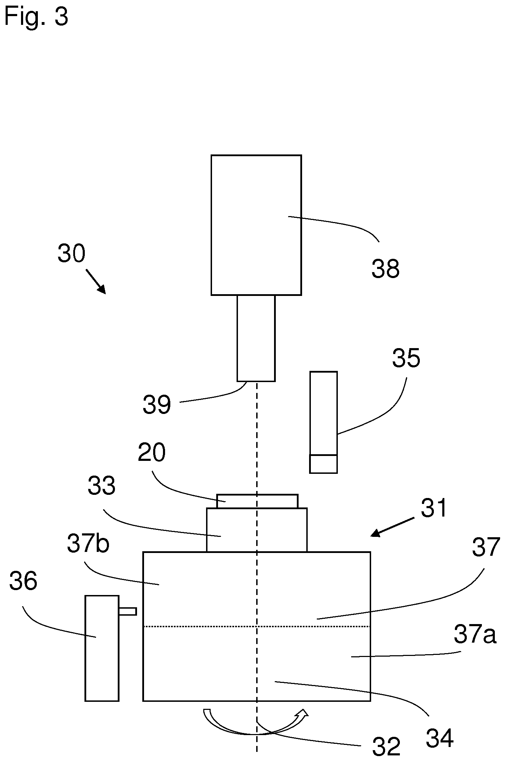

Fig. 3 zeigt schematisch eine Zentrierdrehvorrichtung 30, mit der dieses Verfahren ausführbar ist. Die Zentrierdrehvorrichtung 30 umfasst eine Spindel 31, die um die Drehachse 32 rotierbar ist. Dies ist durch den um die Drehachse 32 angeordneten Pfeil angedeutet. Damit die Rotation möglichst gleichmäßig erfolgt, umfasst die Spindel 31 eine in Fig. 3 nicht näher dargestellte hydrostatische Lagerung 34. Zudem umfasst die Spindel 31 die Halterung 33, die das zu bearbeitende optische Element 20 hält.

Weiterhin umfasst die Spindel 31 eine Ausrichteeinheit 37, die einen ersten Teil 37a und einen zweiten Teil 37b aufweist, welche in Fig. 3 durch die waagerechte Punkt-Linie getrennt sind. Der erste Teil 37a ist dabei so angeordnet, dass seine Ausrichtung zur Drehachse 32 konstant bleibt. Der zweite Teil 37b ist mit der Halterung 33 verbunden und so auf dem ersten Teil 37a gelagert, dass der zweite Teil 37b gegenüber der Drehachse 32 verkippbar ist. Mittels einer Stoßvorrichtung 36 können Stoßprozesse bzw. Stöße gegen den zweiten Teil 37b der Ausrichteeinheit 37 ausgeführt werden, so dass die Ausrichtung des zweiten Teils 37b, und somit des optischen Elements 20, gegenüber der Drehachse 32 veränderbar ist.

Oberhalb des optischen Elements 20 ist eine Signalerfassungsvorrichtung 38 vorgesehen, die über ein fotosensitives Element 39 verfügt. Mittels der Signalerfassungsvorrichtung 38 lässt sich die Ausrichtung der optischen Achse 22 des optischen Elements 20 gegenüber der Drehachse 32 überprüfen, indem ein Lichtstrahl entlang der Drehachse 32 durch das optische Element 20 in Richtung des fotosensitiven Elements 39 geleitet wird.

Schließlich umfasst die Zentrierdrehvorrichtung 30 eine Abtragungsvorrichtung 35. Diese verwendet als Abtragungselement in der gezeigten Ausführungsform einen Ultrakurzpulslaser mit einer Wellenlänge im Femtosekundenbereich.

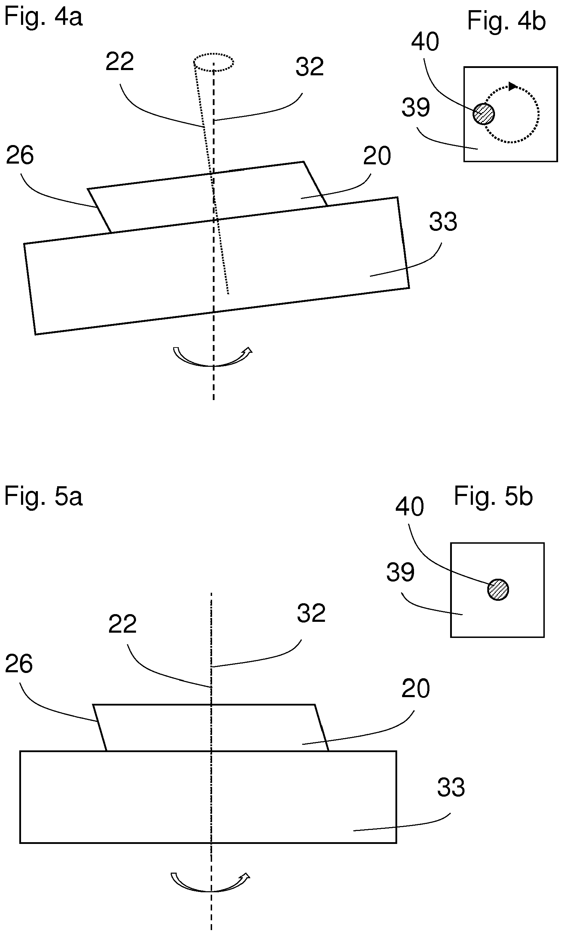

Fig. 4a bis Fig. 6 beschreiben beispielhaft ein erfindungsgemäßes Verfahren zum Vorbereiten eines optischen Elements 20 zum Einsatz in ein optisches System 10 eines Endoskops.

Fig. 4a zeigt schematisch ein optisches Element 20, das von der Halterung 33 gehalten wird. Die optische Achse 22 des optischen Elements 20 stimmt dabei nicht mit der Drehachse 32 der Spindel 31 überein. Bei Rotation der Spindel 31 beschreibt die optische Achse 22 daher eine rotierende Bewegung um die Drehachse 32.

Ein Lichtstrahl, der in der Darstellung in Fig. 4a von unten entlang der Drehachse 32 durch das optische Element 20 geleitet wird, beschreibt auf dem über dem optischen Element 20 angeordneten fotosensitiven Element 40 eine kreisförmige Bewegung, wie in Fig. 4b gezeigt.

Mittels der Stoßvorrichtung 36 und der Ausrichteeinheit 37 wird vor der Abtragung der Mantelfläche 26 die Ausrichtung der optischen Achse 22 des optischen Elements 20 auf der Spindel 31 so verändert, dass die optische Achse 22 des optischen Elements 20 mit der Drehachse 32 der Spindel 31 übereinstimmt, wie in Fig. 5a gezeigt. Fig. 5b stellt dar, wie sich die Ausrichtung der optischen Achse 22 entlang der Drehachse 22 mit der Signalerfassungsvorrichtung 38 überprüfen lässt, da in diesem Fall das Lichtsignal 40 auf dem fotosensitiven Element 39 keine Rotation mehr beschreibt.

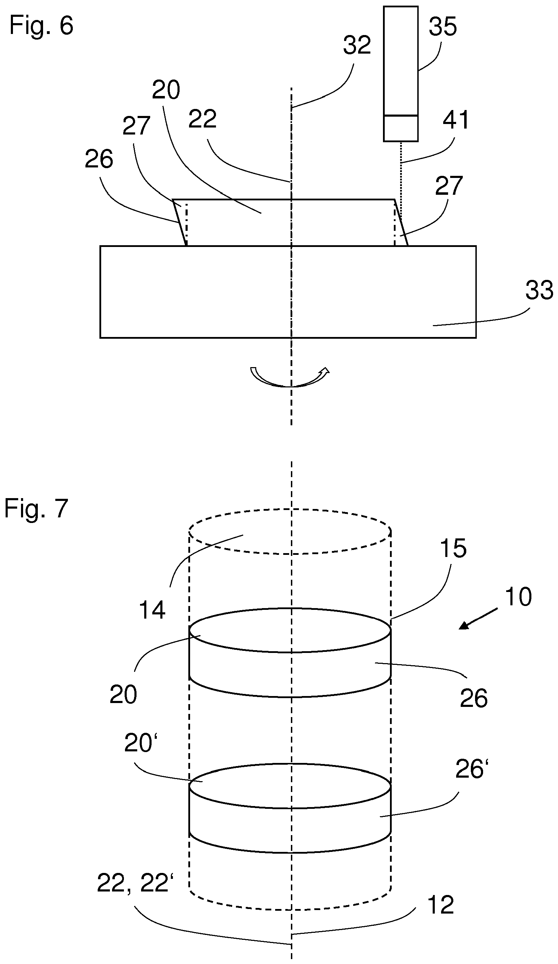

In Fig. 6 ist schematisch der darauffolgende Abtrageprozess gezeigt. Dazu wird die Abtragungsvorrichtung 35 so angeordnet, dass mittels des Ultrakurzpulslasers 41 der äußere Mantelflächenbereich 27 des optischen Elements 20 abgetragen wird.

Mit dem Ultrakurzpulslaser 41 kann direkt das optisch durchlässige Material 28 des optischen Elements 20 abgetragen werden, so dass auf eine Einbettung des optischen Elements 20 in eine Messinghülse verzichtet werden kann.

Die in Fig. 2 und den Fig. 4a bis 6 dargestellte Abweichung der Form des optischen Elements 20 und der Ausrichtung der Achse 22 des optischen Elements 20 ist zur besseren Veranschaulichung stark übertrieben dargestellt.

In Fig. 7 ist schematisch gezeigt, wie zwei optische Elemente 20, 20' in eine Optikfassung 14 des optischen Systems 10 eingesetzt werden. Die Optikfassung 14 des optischen Systems 10 weist dazu eine äußerst präzise gefertigte Innenfläche 15 auf, deren Durchmesser im Wesentlichen dem Durchmesser der optischen Elemente 20, 20' entspricht. Die optischen Elemente 20, 20' werden so in die Optikfassung 14 eingelegt, dass die Mantelflächen 26, 26' der optischen Elemente 20, 20' an der Innenfläche 15 anliegen. Auf diese Weise werden die optischen Elemente 20, 20' so im optischen System 10 ausgerichtet, dass die optischen Achsen 22, 22' der optischen Elemente 20, 20' exakt mit der optischen Achse 12 des optischen Systems 10 übereinstimmen.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 10: optisches System
- 12: optische Achse
- 14: Optikfassung
- 15: Innenfläche
- 16: Hülse
- 20, 20': optisches Element
- 21: Kreis
- 22, 22': optische Achse
- 26, 26': Mantelfläche
- 27: äußerer Mantelflächenbereich
- 28: Material
- 30: Zentrierdrehvorrichtung
- 31: Spindel
- 32: Drehachse
- 33: Halterung
- 34: hydrostatische Lagerung
- 35: Abtragungsvorrichtung
- 36: Stoßvorrichtung
- 37: Ausrichteeinheit
- 37a: erster Teil
- 37b: zweiter Teil
- 38: Signalerfassungsvorrichtung
- 39: fotosensitives Element
- 40: Lichtsignal
- 41: Ultrakurzpulslaser

## Patentansprüche

1. Verfahren zum Vorbereiten eines optischen Elements (20, 20') zum Einsatz in ein optisches System (10) eines Endoskops, wobei das optische Element (20, 20') eine optische Achse (22, 22') und eine zur optischen Achse (22, 22') im Wesentlichen parallele Mantelfläche (26, 26') aufweist, mit den folgenden Verfahrensschritten:
- Anordnen des optischen Elements (20, 20') in einer Halterung (33) einer Spindel (31), die das optische Element (20, 20') um eine Drehachse (32) der Spindel (31) rotiert,
- Ausrichten des optischen Elements (20, 20') auf der Spindel (31), so dass die optische Achse (22, 22') des optischen Elements (20, 20') mit der Drehachse (32) der Spindel (31) übereinstimmt,
- Abtragen eines äußeren Mantelflächenbereichs (27) des optischen Elements (20, 20'), bis die Mantelfläche (26, 26') einen konstanten Abstand zur optischen Achse (22, 22') des optischen Elements (20, 20') aufweist,
**dadurch gekennzeichnet, dass** das Abtragen des äußeren Mantelflächenbereichs (27) durch Laserablation, insbesondere mittels eines Ultrakurzpulslasers (41), erfolgt.

2. Verfahren nach Anspruch 1, **dadurch gekennzeichnet, dass** das optische Element (20, 20') aus einem optisch durchlässigen Material (28) gefertigt wird, wobei beim Abtragen des äußeren Mantelflächenbereichs (27) direkt das optisch durchlässige Material (28) abgetragen wird.

3. Verfahren nach Anspruch 1 oder 2, **dadurch gekennzeichnet, dass** das Ausrichten des optischen Elements (20, 20') auf der Spindel (31) mittels wenigstens einer Stoßvorrichtung (36) erfolgt, wobei die Stoßvorrichtung (36) durch Stöße mit einer mit der Halterung (33) verbundenen Ausrichteeinheit (37) die Ausrichtung der optischen Achse (22, 22') des optischen Elements (20, 20') bezüglich der Drehachse (32) der Spindel (31) verändert.

4. Verfahren nach einem der Ansprüche 1 bis 3, **dadurch gekennzeichnet, dass** das Ausrichten des optischen Elements (20, 20') auf der Spindel (31) durch Erfassung eines Lichtsignals (40) auf einem fotosensitiven Element (39) einer Signalerfassungsvorrichtung (38) überprüft wird, wobei das Lichtsignal (39) erzeugt wird, indem ein Lichtstrahl durch das optische Element (20, 20') entlang der Drehachse (32) der Spindel (31) in Richtung des fotosensitiven Elements (39) geleitet wird, wobei die Ausrichtung des optischen Elements (20, 20') so lange verändert wird, bis bei einer Drehung der Spindel (31) eine Position des Lichtsignals (40) auf dem fotosensitiven Element (39) konstant bleibt.

5. Verfahren zum Zentrieren eines optischen Elements (20, 20') in einem optischen System (10) eines Endoskops, wobei das optische System (10) eine optische Achse (12) aufweist und das optische Element (20, 20') eine optische Achse (22, 22') und eine zur optischen Achse (22, 22') im Wesentlichen parallele Mantelfläche (26, 26') aufweist, mit den folgenden Verfahrensschritten:
- Abtragen eines äußeren Mantelflächenbereichs (27) des optischen Elements, bis die Mantelfläche (26, 26') einen konstanten Abstand zur optischen Achse (22, 22') des optischen Elements (20, 20') aufweist,
- Einsetzen des optischen Elements (20, 20') in das optische System (10), wobei die Mantelfläche (26, 26') des optischen Elements (20, 20') an einer Innenfläche (15) einer Optikfassung (14) des optischen Systems (10) anliegt und die optische Achse (22, 22') des optischen Elements (20, 20') mit der optischen Achse (12) des optischen Systems (10) übereinstimmt,
**dadurch gekennzeichnet, dass** das Abtragen des äußeren Mantelflächenbereichs (27) des optischen Elements (20, 20') mittels Laserablation erfolgt, insbesondere mittels eines Verfahrens nach einem der Ansprüche 1 bis 4.

6. Verfahren nach Anspruch 5, **dadurch gekennzeichnet, dass** mehrere optische Elemente (20, 20') in das optische System (10) eingesetzt werden, wobei die optischen Achsen (22, 22') aller optischen Elemente (20, 20') mit der optischen Achse (12) des optischen Systems (10) in Übereinstimmung gebracht werden und wobei die Mantelflächen (26, 26') aller optische Elemente (20, 20') an der Innenfläche (15) der Optikfassung (14) des optischen System (10) anliegen.

7. Zentrierdrehvorrichtung (30), umfassend eine um eine Drehachse (32) rotierbare Spindel (31) mit einer Halterung (33) für ein optisches Element (20, 20') für ein Endoskop und eine Abtragungsvorrichtung (35) mit einem Abtragungselement, das dazu eingerichtet ist, eine Mantelfläche (26, 26') eines optischen Elements (20, 20') in der Halterung (33) abzutragen, **dadurch gekennzeichnet, dass** das Abtragungselement ein Ultrakurzpulslaser (41) ist.

8. Zentrierdrehvorrichtung (30) nach Anspruch 7, **dadurch gekennzeichnet, dass** wenigstens eine Stoßvorrichtung (36) umfasst ist, die dazu eingerichtet ist, durch Stöße mit einer mit der Halterung (33) verbundenen Ausrichteeinheit (37) die Ausrichtung der Halterung (33) bezüglich der Drehachse (32) zu verändern.

9. Zentrierdrehvorrichtung (30) nach Anspruch 7 oder 8, **dadurch gekennzeichnet, dass** wenigstens eine Signalerfassungsvorrichtung (38) mit einem fotosensitiven Element (39) umfasst ist, wobei das fotosensitive Element (39) so angeordnet ist, dass durch die Signalerfassungsvorrichtung (38) ein Lichtsignal (40) eines entlang der Drehachse (32) der Spindel (31) geleiteten Lichtstrahls erfassbar ist.

10. Zentrierdrehvorrichtung (30) nach einem der Ansprüche 7 bis 10, **dadurch gekennzeichnet, dass** die Spindel (31) eine hydrostatische Lagerung (34) aufweist.

11. Endoskop mit einem optischen Element (20, 20') zum Einsatz in ein optisches System (10) des Endoskops, wobei das optische Element (20, 20') eine optische Achse (22, 22') und eine zur optischen Achse (22, 22') im Wesentlichen parallele Mantelfläche (26, 26') aufweist und wobei das optische Element (20, 20') mit einem Verfahren nach einem der Ansprüche 1 bis 4 vorbereitet ist.

12. Endoskop mit einem optischen Element (20, 20') zum Einsatz in ein optisches System (10) des Endoskops, wobei das optische Element (20, 20') eine optische Achse (22, 22') und eine zur optischen Achse (22, 22') im Wesentlichen parallele Mantelfläche (26, 26') aufweist und wobei das optische Element (20, 20') mit einem Verfahren nach Anspruch 5 oder 6 zentriert ist.

## Claims

1. A method for preparing an optical element (20, 20') for insertion into an optical system (10) of an endoscope, wherein the optical element (20, 20') has an optical axis (22, 22') and a peripheral surface (26, 26') that is basically parallel to the optical axis (22, 22'), with the following method steps:
- Arranging the optical element (20, 20') in a mounting (33) of a spindle (31) which rotates the optical element (20, 20') about an axis of rotation (32) of the spindle (31),
- Aligning the optical element (20, 20') on the spindle (31) such that the optical axis (22, 22') of the optical element (20, 20') coincides with the axis of rotation (32) of the spindle (31),
- Removing an outer peripheral surface region (27) of the optical element (20, 20') until the peripheral surface (26, 26') has a constant spacing from the optical axis (22, 22') of the optical element (20, 20'),
**characterized in that** the removal of the outer peripheral surface region (27) takes place by laser ablation, in particular by means of an ultra-short pulse laser (41).

2. The method according to claim 1, **characterized in that** the optical element (20, 20') is made of an optically permeable material (28), wherein the optically permeable material (28) is removed directly during the removal of the outer peripheral surface region (27).

3. The method according to claim 1 or 2, **characterized in that** the alignment of the optical element (20, 20') on the spindle (31) takes place by means of at least one impact device (36), wherein the impact device (36) changes the alignment of the optical axis (22, 22') of the optical element (20, 20') with respect to the axis of rotation (32) of the spindle (31) through impacts with an alignment unit (37) connected to the mounting (33).

4. The method according to one of claims 1 to 3, **characterized in that** the alignment of the optical element (20, 20') on the spindle (31) is checked by detecting a light signal (40) on a photosensitive element (39) of a signal detection device (38), wherein the light signal (39) is generated **in that** a light beam is conducted through the optical element (20, 20') along the axis of rotation (32) of the spindle (31) in the direction of the photosensitive element (39), wherein the alignment of the optical element (20, 20') is changed until a position of the light signal (40) on the photosensitive element (39) remains constant when the spindle (31) is rotated.

5. A method for centering an optical element (20, 20') in an optical system (10) of an endoscope, wherein the optical system (10) has an optical axis (12) and the optical element (20, 20') has an optical axis (22, 22') and a peripheral surface (26, 26') that is basically parallel to the optical axis (22, 22'), with the following method steps:
- Removing an outer peripheral surface region (27) of the optical element until the peripheral surface (26, 26') has a constant spacing from the optical axis (22, 22') of the optical element (20, 20'),
- Inserting the optical element (20, 20') into the optical system (10), wherein the peripheral surface (26, 26') of the optical element (20, 20') abuts an inner surface (15) of an optical frame (14) of the optical system (10) and the optical axis (22, 22') of the optical element (20, 20') coincides with the optical axis (12) of the optical system (10),
**characterized in that** the removal of the outer peripheral surface region (27) of the optical element (20, 20') takes place by means of laser ablation, in particular by means of a method according to one of claims 1 to 4.

6. The method according to claim 5, **characterized in that** multiple optical elements (20, 20') are inserted into the optical system (10), wherein the optical axes (22, 22') of all the optical elements (20, 20') are brought to coincide with the optical axis (12) of the optical system (10) and wherein the peripheral surfaces (26, 26') of all the optical elements (20, 20') abut the inner surface (15) of the optical frame (14) of the optical system (10).

7. A centering and rotating device (30) comprising a spindle (31) that can be rotated about an axis of rotation (32) with a mounting (33) for an optical element (20, 20') for an endoscope and a removal device (35) with a removal element which is configured to remove a peripheral surface (26, 26') of an optical element (20, 20') in the mounting (33), **characterized in that** the removal element is an ultra-short pulse laser (41).

8. The centering and rotating device (30) according to claim 7, **characterized in that** at least one impact device (36) is comprised which is configured to change the alignment of the mounting (33) with respect to the axis of rotation (32) through impacts with an alignment unit (37) connected to the mounting (33).

9. The centering and rotating device (30) according to claim 7 or 8, **characterized in that** at least one signal detection device (38) with a photosensitive element (39) is comprised, wherein the photosensitive element (39) is arranged such that a light signal (40) of a light beam conducted along the axis of rotation (32) of the spindle (31) can be detected by the signal detection device (38).

10. The centering and rotating device (30) according to one of claims 7 to 10, **characterized in that** the spindle (31) has a hydrostatic bearing (34).

11. An endoscope with an optical element (20, 20') for insertion into an optical system (10) of the endoscope, wherein the optical element (20, 20') has an optical axis (22, 22') and a peripheral surface (26, 26') that is basically parallel to the optical axis (22, 22') and wherein the optical element (20, 20') is prepared with a method according to one of claims 1 to 4.

12. The endoscope with an optical element (20, 20') for insertion into an optical system (10) of the endoscope, wherein the optical element (20, 20') has an optical axis (22, 22') and a peripheral surface (26, 26') that is basically parallel to the optical axis (22, 22') and wherein the optical element (20, 20') is centered with a method according to claim 5 or 6.

## Revendications

1. Procédé de préparation d'un élément optique (20, 20') destiné à être inséré dans un système optique (10) d'un endoscope, l'élément optique (20, 20') présentant un axe optique (22, 22') et une surface périphérique (26, 26') sensiblement parallèle à l'axe optique (22, 22'), comprenant les étapes de procédé suivantes :
- disposition de l'élément optique (20, 20') dans un support (33) d'une broche (31) qui fait tourner l'élément optique (20, 20') autour d'un axe de rotation (32) de la broche (31),
- alignement de l'élément optique (20, 20') sur la broche (31), de sorte que l'axe optique (22, 22') de l'élément optique (20, 20') coïncide avec l'axe de rotation (32) de la broche (31),
- ablation d'une zone extérieure (27) de surface périphérique de l'élément optique (20, 20') jusqu'à ce que la surface périphérique (26, 26') présente une distance constante par rapport à l'axe optique (22, 22') de l'élément optique (20, 20'),
**caractérisé en ce que** l'ablation de la zone extérieure (27) de surface périphérique est effectué par ablation au laser, en particulier au moyen d'un laser (41) à impulsions ultracourtes.

2. Procédé selon la revendication 1, **caractérisé en ce que** l'élément optique (20, 20') est fabriqué à partir d'un matériau optiquement transparent (28), le matériau optiquement transparent (23) étant directement enlevé lors de l'ablation de la zone extérieure (27) de surface périphérique.

3. Procédé selon la revendication 1 ou la revendication 2, **caractérisé en ce que** l'alignement de l'élément optique (20, 20') sur la broche (31) s'effectue au moyen d'au moins un dispositif à chocs (36), le dispositif à chocs (36) modifiant, par des chocs au moyen d'une unité d'alignement (37) reliée au support (33), l'alignement de l'axe optique (22, 22') de l'élément optique (20, 20') par rapport à l'axe de rotation (32) de la broche (31).

4. Le procédé selon l'une des revendications 1 à 3, **caractérisé en ce que** l'on vérifie l'alignement de l'élément optique (20, 20') sur la broche (31) en détectant un signal lumineux (40) sur un élément photosensible (39) d'un dispositif (38) d'acquisition de signal, le signal lumineux (39) étant généré en faisant passer un faisceau lumineux à travers l'élément optique (20, 20') le long de l'axe de rotation (32) de la broche (31) en direction de l'élément photosensible (39), l'alignement de l'élément optique (20, 20') étant modifiée jusqu'à ce que, lors d'une rotation de la broche (31), une position du signal lumineux (40) sur l'élément photosensible (39) reste constante.

5. Procédé de centrage d'un élément optique (20, 20') dans un système optique (10) d'un endoscope, le système optique (10) présentant un axe optique (12) et l'élément optique (20, 20') présentant un axe optique (22, 22') et une surface périphérique (26, 26') sensiblement parallèle à l'axe optique (22, 22'), comprenant les étapes de procédé suivantes :
- ablation d'une zone extérieure (27) de surface périphérique de l'élément optique jusqu'à ce que la surface périphérique (26, 26') présente une distance constante par rapport à l'axe optique (22, 22') de l'élément optique (20, 20'),
- insertion de l'élément optique (20, 20') dans le système optique (10), la surface périphérique (26, 26') de l'élément optique (20, 20') étant en contact avec une surface intérieure (15) d'une monture optique (14) du système optique (10) et l'axe optique (22, 22') de l'élément optique (20, 20') coïncidant avec l'axe optique (12) du système optique (10),
**caractérisé en ce que** l'ablation de la zone extérieure (27) de surface périphérique de l'élément optique (20, 20') est réalisée par ablation au laser, notamment au moyen d'un procédé selon l'une des revendications 1 à 4.

6. Procédé selon la revendication 5, **caractérisé en ce que** plusieurs éléments optiques (20, 20') sont insérés dans le système optique (10), les axes optiques (22, 22') de tous les éléments optiques (20, 20') étant mis en correspondance avec l'axe optique (12) du système optique (10) et les surfaces périphériques (26, 26') de tous les éléments optiques (20, 20') étant en appui sur la surface intérieure (15) de la monture optique (14) du système optique (10).

7. Dispositif rotatif de centrage (30), comprenant une broche (31) pouvant tourner autour d'un axe de rotation (32) avec un support (33) pour un élément optique (20, 20') pour un endoscope et un dispositif d'ablation (35) avec un élément d'ablation qui est conçu pour ablater une surface périphérique (26, 26') d'un élément optique (20, 20') dans le support (33), **caractérisé en ce que** l'élément d'ablation est un laser (41) à impulsions ultracourtes.

8. Dispositif rotatif de centrage (30) selon la revendication 7, **caractérisé en ce qu'**il comprend au moins un dispositif à chocs (36) agencé pour modifier l'alignement du support (33) par rapport à l'axe de rotation (32) par des chocs au moyen d'une unité d'alignement (37) reliée au support (33).

9. Dispositif rotatif de centrage (30) selon la revendication 7 ou la revendication 8, **caractérisé en ce qu'**il comprend au moins un dispositif (38) de détection de signal comportant un élément photosensible (39), l'élément photosensible (39) étant agencé de manière à permettre la détection par le dispositif (38) de détection de signal d'un signal lumineux (40) d'un faisceau lumineux guidé le long de l'axe de rotation (32) de la broche (31).

10. Dispositif rotatif de centrage (30) selon l'une des revendications 7 à 10, **caractérisé en ce que** la broche (31) comporte un palier hydrostatique (34).

11. Endoscope comprenant un élément optique (20, 20') destiné à être inséré dans un système optique (10) de l'endoscope, l'élément optique (20, 20') présentant un axe optique (22, 22') et une surface périphérique (26, 26') sensiblement parallèle à l'axe optique (22, 22'), l'élément optique (20, 20') étant préparé par un procédé selon l'une des revendications 1 à 4.

12. Endoscope comprenant un élément optique (20, 20') destiné à être inséré dans un système optique (10) de l'endoscope, l'élément optique (20, 20') présentant un axe optique (22, 22') et une surface périphérique (26, 26') sensiblement parallèle à l'axe optique (22, 22'), et l'élément optique (20, 20') étant centré par un procédé selon la revendication 5 ou la revendication 6.
